# EUROPEAN PATENT APPLICATION

(11) **EP 0 558 039 A2**
(43) Date of publication of application: **01.09.1993**
(21) Application number: 93103063.9
(22) Date of filing: 26.02.1993
(51) Int. Cl.: A61B 17/39, A61M 25/01

(54) **Electro-ligator and valvulotomy system therefor**

(30) Priority: 28.02.1992 US 843634; 08.06.1992 US 895090; 19.02.1993 US 19772; 22.02.1993 US 20631
(71) Applicant: ENDOVASCULAR, INC., Costa Mesa, California 92626 (US)
(72) Inventor: Cohen, Donald, Irvine, California 92714 (US); Kumm, Lance, Tustin, California 92680 (US)
(74) Representative: VOSSIUS & PARTNER

(57) **Abstract**

A device (70) for closing off a side branch of a human body vessel, such as a saphenous vein, includes a metal core (73) which has a distal segment (75), a distal end (84), and a proximal segment (74). An electrically insulative sheath (77) is shrink-wrapped around a substantial portion of the proximal segment (74) of the core (73) to electrically insulate the core (73). Additionally, a metallic coil (79) is disposed around and bonded to a substantial portion of the distal segment (75) of the core (73). To energize the coil (79) and the bonding material (83) when the device (70) has been positioned as appropriate within the blood vessel, a source (72) of electricity is electrically connected to the core (73), and the source (72) of electricity can be activated to selectively energize the core (73) and, hence, the coil (79). Thereby, a thrombosis is induced in the blood vessel and the vessel walls are dessicated, such that the blood vessel is substantially reduced and occluded. If desired, the device (70) can be slidably engaged with a catheter-like device, e.g., an endoscopic valvulotome.

## Description

The present invention relates generally to surgical tools for reducing leakage from blood vessels during surgery, and more particularly to electric coagulators.

When surgery is performed on a patient, particularly surgery requiring a detailed, unimpeded view of the surgery site, it is necessary to keep the site free from fluid and debris which could otherwise interfere with the surgeons's view of the surgery site. Such fluid and debris can not only impede the surgeon's view of the site, but also can potentially infect the site.

Despite the use of well-known techniques, e.g., the application of tourniquets, to stem the flow of bodily fluids into the surgery site, however, small sources of fluid flow can still periodically cause fluids such as blood to flow into a surgery site during surgery. To avoid this, devices have been developed for burning these small sources of fluid and thereby coagulating blood and tissue around the sources.

Typically, these coagulating devices include a tool which can be positioned adjacent the source of bleeding and then rapidly heated, e.g., by electricity or laser energy, to coagulate blood and tissue surrounding the source of bleeding.

One well-known source of electrical coagulating pulses that can be conducted to a coagulating tool is the Bovie model pulse generator manufactured by Valley Laboratories of Colorado. The Bovie generator is typically connected via an electrical line to a metallic coagulator which has been positioned adjacent a source of bleeding. The Bovie generator can then be activated by the surgeon to produce an electrical pulse to heat the coagulator and stop blood flow from the source of bleeding.

Unfortunately, many, if not most, coagulators are relatively bulky devices which are designed for use within a relatively large body cavity that has been exposed by surgery, and which therefore cannot easily be used to stop blood flow from "hard-to-reach" sources. In other words, existing coagulators cannot easily be used to stop the flow of blood from sources that may not be located within the body cavity which has been exposed by surgery, but which instead are located in relatively narrow "side branches" of body vessels near the operating site.

For example, when performing endoscopic valvulotomy on a human saphenous vein incident to in situ saphenous vein bypass surgery, side branches of the saphenous vein must be ligated. Ligating the side branches permits the saphenous vein to be arterialized, i.e., permits the saphenous vein to be anastamosed to an artery at locations on the artery which are proximal and distal to an occluded segment of the artery to bypass the occluded segment, without risking invasion of the venous system by arterial blood. More particularly, by ligating the side branches, arterial blood which is permitted to flow through the saphenous vein and thereby bypass the occluded segment of the artery is prevented from invading the venous system through the side branches.

Accordingly, methods have been developed for stopping the flow of blood from side branches by constricting the particular side branch, i.e., by ligating the side branch with surgical line. Unfortunately, ligating body vessels can be time-consuming and labor-intensive, compared with stopping blood flow by coagulating. Also, ligating side branches can be harmful to the side branches.

It is therefore an object of the present invention to provide a side branch coagulator that can coagulate blood and tissue in narrow body vessels, and particularly in blood vessels, to stop blood flow from sources of bleeding located in the body vessels. It is a further object of the present invention to provide a side branch coagulator that can be used in conjunction with existing cauterising current generators. Yet another object of the present invention is to provide a side branch coagulator that is easy to use and cost-effective to manufacture. Still another object of the present invention is to provide an electro-ligator for occluding side branches of a human saphenous vein during in situ saphenous vein bypass surgery.

A side branch coagulator in accordance with the present invention includes an electrically conductive wire which is connectable to a source of electricity. The coagulator can be advanced into relatively narrow body vessel side branches containing sources of bleeding to coagulate blood flowing from the particular side branch. Also, the coagulator includes an electrically resistive heat generator which is attached to a distal end of the wire and which is heated when electricity is conducted through the wire. Further, the coagulator includes guide means surrounding the wire for selectively exposing the heat generator to relatively narrow side branches to coagulate blood flowing from the side branch. Accordingly, the coagulator of the present invention can be efficaciously used to stop the flow of bodily fluids from sources of bleeding that are located in relatively narrow side branches of body vessels.

In another aspect of the present invention, the coagulator includes an electrically-conductive insulated wire and an electrically-resistive ball welded or soldered to a distal end of the wire. The ball can be positioned inside a narrow side branch that contains a source of internal bleeding, and current can be passed through the wire to the ball to heat the ball and coagulate the side branch. Alternatively, the ball can be positioned directly against a source of bleeding against a source of bleeding, and current can be passed through the wire to the ball to cauterize the source of bleeding. The wire and ball both have comparatively small diameters, so that the wire and ball can be positioned within relatively narrow "side branches" of body vessels to coagulate blood flowing out of the particular side branch into the operating site.

To advance the wire with ball to the source of internal bleeding, the wire with ball is slidably engaged with the lumen of a suitable guide catheter, e.g., the cutting catheter of the device disclosed in U.S. Patent No. 5,026,383, owned by the assignee who owns the present invention. Accordingly, the ball can be retracted into the catheter to house the ball when the coagulator is not being used to cauterize a source of bleeding or coagulate a side branch. Also, the wire can be slid distally within the catheter to expose the ball when it is desired to neutralize the source of bleeding.

The wire also has a proximal end, and the proximal end is electrically connected to a source of electric current, e.g., a Bovie model current source manufactured by Valley Laboratories of Colorado. A collet having a plurality of flexible leaves is preferably connected to the proximal end of the guide catheter and is coaxial with the guide catheter. The leaves of the collet are materially biased outwardly, and a resilient O-ring is attached to the leaves of the collet. The O-ring circumscribes the interior surface of the collet, and the insulated wire extends through the O-ring, collet, and catheter.

A chuck surrounds the collet and is slidably engaged with the collet. The chuck has a "hold" position relative to the collet, wherein the chuck urges the leaves of the collet inwardly to cause the O-ring to grip the wire to hold the wire stationary with respect to the collet and catheter. Also, the chuck can be moved from the hold position to a "slide" position, wherein the chuck releases the leaves of the collet to permit the wire to slide relative to the collet and catheter.

Accordingly, the ball of the coagulator can be housed within the guide catheter near the distal end of the guide catheter by moving the chuck to the slide position and manually sliding the wire as appropriate relative to the catheter. Then, the chuck is moved to the hold position, the catheter advanced into a body vessel, and the distal end of the catheter positioned adjacent a source of bleeding. Next, when it is desired to stop the source of bleeding, the chuck is moved to the slide position and the rigid wire is slid distally to position the ball and the distal segment of the rigid wire in the side branch. The current generator is then activated to cause a pulse of current to traverse the wire and heat the ball. This causes a thrombus to form in the side branch, which thereby coagulates the side branch.

If desired, in an alternative embodiment the distal segment of the wire can be materially biased in an arcuate configuration. In other words, the distal segment of the wire is normally curved. When the distal segment is proximally retracted into the guide catheter, the walls of the catheter urge against the distal segment to cause the distal segment to straighten sufficiently to permit housing the distal segment within the catheter. On the other hand, when the distal segment of the wire is slid out of the guide catheter, the distal segment of the wire resumes its preformed arcuate shape, permitting the ball to be efficaciously positioned near hard-to-reach sources of bleeding. Stated differently, when the distal segment of the wire is free of the guide catheter, the distal segment springs into a curved shape.

In another aspect of the alternate embodiment, a coagulating probe is provided which has a coagulating element attached to the probe. A tubular catheter defining a longitudinal axis and having a distal end is disposed in surrounding relationship with the probe, such that the probe is disposed within the catheter to permit extending the coagulating element distally beyond the distal end of the catheter. In the alternative embodiment, the probe is configured so that the element projects laterally beyond a side of the tubular catheter when the element is extended distally beyond the distal end of the catheter.

In this alternative embodiment, the probe is a resilient member which reacts in response to axial movement of the probe within the catheter to urge the coagulating element towards the longitudinal axis of the catheter. This permits the coagulating element to be withdrawn into the catheter. Preferably, the resilient member is a wire which is resilient in the sense that the distal segment of the wire is materially biased in an arcuate configuration, such that the distal segment of the wire can be urged into the catheter and, when slid out of the catheter, will resiliently resume the arcuate configuration in which the wire is biased.

In yet another alternative embodiment of the present invention, an electro-ligator includes an elongated bendable metal core which has a distal segment, a distal end, and a proximal segment, and the core can be advanced into a human blood vessel. An electrically insulative sheath is shrink-wrapped around a substantial portion of the proximal segment of the core to electrically insulate the core. Additionally, a metallic coil is disposed around a substantial portion of the distal segment of the core, and a bonding material having low electrical resistance may, if desired, be deposited on a substantial portion of the distal end of the core to bond the coil to the core. To energize the coil and the bonding material, a source of electricity is electrically connected to the core, and the source of electricity can be activated to selectively energize the core and, hence, the coil and the bonding material. Thereby, a thrombosis is induced in the blood vessel, and the blood vessel wall is dessicated locally, such that fluid flow through the blood vessel is stopped. In other words, the blood vessel is cauterized to stop fluid flow through the vessel.

In a first embodiment, the distal segment of the core is straight, whereas in a second embodiment, the distal segment of the core is biased into a predetermined arcuate shape. Arcuate shapes that may be used for the distal segment include a "J" and an "S".

Preferably, to augment the flexibility of the distal segment of the core, the diameter of the distal segment of the core is less than the diameter of the proximal segment of the core. Also, the sheath is sufficiently thick and sufficiently pure to ensure that less than about three-tenths of a milliampere (0.3ma) flows through the sheath when at least twelve hundred volts (1200V) is applied across the sheath. If desired, an outer coil may be disposed around the sheath and connected to the source of electricity to establish a bipolar electro-cautery element.

In another aspect of the electro-ligator of the present invention, a device for cauterizing a side branch of a blood vessel includes a source of electricity and an electrical conductor which is electrically connected to the source of electricity. As intended by the present invention, the electrical conductor has a distal segment that is biased into a predetermined configuration to facilitate advancing the distal segment into the side branch. Also, the electrical conductor has a proximal segment, and an electrically resistive sheath surrounds the proximal segment of the electrical conductor. Further, an electro-cautery element which includes at least one coil is attached to the distal segment of the electrical conductor for becoming sufficiently energized when the source of electricity is activated to transmit electricity through the conductor to the electro-cautery element. Thereby, a narrowing and a thrombosis are induced in the side branch of the blood vessel to block fluid flow through the side branch.

In yet another aspect of the alternate embodiment of the present invention, a method is disclosed for cauterizing a side branch of a human saphenous vein to substantially stop fluid flow through the side branch. The method of the present invention includes the steps of providing a device which has an electrical conductor. The electrical conductor of the device has a distal segment which is biased into a predetermined configuration to facilitate advancing the distal segment into the side branch, and a proximal segment. An electrically resistive sheath surrounds the proximal segment of the electrical conductor, and an electro-cautery element is attached to the distal segment of the electrical conductor for becoming energized when electricity is applied to it.

In accordance with the method disclosed herein, the conductor of the device is electrically connected to a source of electricity. Then, at least the electro-cautery element of the device is advanced into the side branch of the saphenous vein. The source of electricity is then activated to energize the electro-cautery element to cauterize the side branch and thereby substantially block fluid flow through the side branch. Consequently, blood flow in the side branch to or from the saphenous vein is substantially stopped.

In the preferred embodiment, the electro-ligator can be slidably engaged with the lumen of a catheter-like apparatus, such as a valvulotome for the in-situ cutting of venous valves. The valvulotome of the present invention has a catheter with axial column strength sufficient for pushing the catheter through a vein. An optical fiber is disposed in the catheter, and a cutting head is bonded with epoxy to the catheter.

The cutting head has a pair of cutting blades and a light passageway is established between the blades. As intended by the present invention, the optical fiber is in light communication with the light passageway for collecting light therefrom and directing the light to a display apparatus for displaying a video image of at least a portion of the light passageway.

The details of the present invention, both as to its construction and operation, can best be understood in reference to the accompanying drawings, in which like numerals refer to like parts, and in which:
Figure 1 is a perspective view of the side branch coagulator of the present invention, shown in one intended environment;
Figure 2 is a side view of the side branch coagulator of the present invention, shown within a body vessel with the ball positioned adjacent a source of bleeding, with portions of the body vessel broken away;
Figure 3 is a perspective view of the distal segment of one embodiment of the rigid wire and ball of the side branch coagulator of the present invention, with the extreme distal portion of the insulation broken away for clarity;
Figure 4 is a perspective view of another embodiment of the distal segment of the rigid wire and ball of the side branch coagulator of the present invention, showing a wire having a distal segment materially biased into a preformed arcuate shape, with the extreme distal portion of the insulation broken away for clarity;
Figure 5 is a perspective view of the side branch coagulator of the present invention, with the ball retracted to its housed position within the guide catheter, with portions broken away;
Figure 6 is a cross-sectional view of the side branch coagulator of the present invention, as seen along the line 6-6 in Figure 5;
Figure 7 is a cross-sectional view of the side branch coagulator of the present invention, as would be seen along the line 6-6 in Figure 5, with the ball slid out of the guide catheter;
Figure 8 is a perspective view of an alternate embodiment of the present invention, shown in operative engagement with a valvulotome, with some elements shown schematically;
Figure 9 is a cross-sectional view of the device shown in Figure 8, as seen along the line 9-9 in Figure 8, with portions broken away;
Figure 10 is a cross-sectional view of an alternate embodiment of the device shown in Figure 8, as would be seen along the line 10-10 in Figure 8, with portions broken away;
Figure 11 is a cross-sectional view of an alternate embodiment of the device shown in Figure 8, as would be seen along the line 11-11 in Figure 8, with portions broken away;
Figure 12 is a cross-sectional view of an alternate embodiment of the device shown in Figure 8, as would be seen along the line 12-12 in Figure 8, with portions broken away;
Figure 13 is a cross-sectional view of an alternate embodiment of the device shown in Figure 8, as would be seen along the line 13-13 in Figure 8, with portions broken away;
Figure 14 is a cross-sectional view of the valvulotome shown in Figure 8, as seen along the line 14-14 in Figure 8;
Figure 15 is a cross-sectional view of the valvulotome shown in Figure 8, as seen along the line 15-15 in Figure 8;
Figure 16 is a cross-sectional view of the valvulotome shown in Figure 8, as seen along the line 16-16 in Figure 8;
Figure 17 is a cross-sectional view of the valvulotome shown in Figure 8, as seen along the line 17-17 in Figure 8; and
Figure 18 is a cross-sectional view of an alternate embodiment of the valvulotome shown in Figure 8, as would be seen along the line 18-18 in Figure 8, showing another configuration of the working channel.

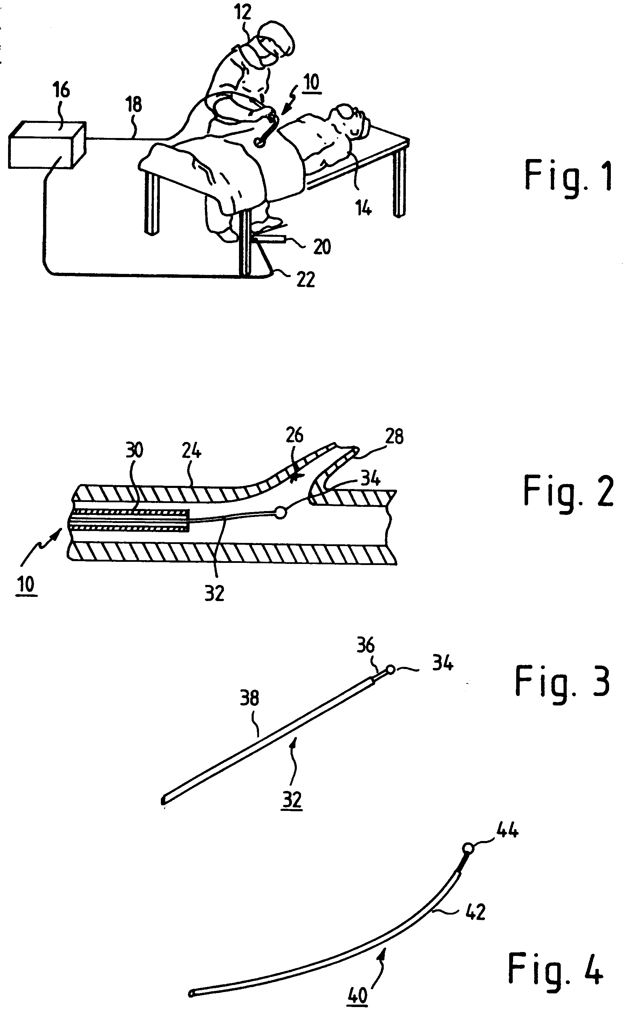

Referring initially to Figure 1, a side branch coagulator, generally designated 10, is shown being used by a surgeon 12 incident to surgery on a patient 14 to stop the flow of blood from a source of bleeding in a vessel of the patient 14. The vessel shown in Figure 1 is a blood vessel. While Figure 1 shows that the side branch coagulator 10 is being used in conjunction with thigh surgery, it is to be understood that the coagulator 10 can be used in other types of surgery, e.g., chest surgery or neurosurgery. Also, the side branch coagulator 10 can be used to stop the flow of blood in segments of leg veins which have had their valves removed prefatory to transplanting the leg veins in other parts of the body, e.g., during in situ endoscopic saphenous vein bypass surgery.

As further shown in Figure 1, the side branch coagulator 10 is connected to a source 16 of electrical pulses via an electrical line 18. The source 16 of electrical pulses can be any suitable source of pulses which is appropriate for generating an electrical current pulse in the side branch coagulator 10 to cause thrombi to form in side branches of the patient 14 to coagulate the side branches. For example, the source of electrical pulses 16 can be a Bovie brand generator manufactured by Valley Laboratories of Colorado.

Figure 1 shows that the surgeon 12 may activate the source 16 of electrical pulses to generate electrical pulses by depressing a foot pedal 20 which is electrically connected via an electrical line 22 to the source 16. Specifically, the surgeon 12 can position the side branch coagulator 10 as appropriate for coagulating a side branch within the patient 14. Then, the surgeon 12 steps on the foot pedal 20 to cause the source 16 of electrical pulses to generate an electrical pulse to traverse the coagulator 10 and thereby cause a thrombus to form in side branch. It is to be understood that the coagulator 10 can also be used to cauterize sources of bleeding within the patient 14.

Now referring to Figure 2, the side branch coagulator 10 can be advanced through an artery 24 of the patient 14 and positioned near a source 26 of bleeding that exists in a side branch 28 of the artery 24. Specifically, the side branch coagulator 10 includes a tubular guide catheter 30, a probe which, in the preferred embodiment, is an elongated, substantially rigid wire 32, and a coagulating element which is preferably a ball 34, and the side branch coagulator 10 is advanced through the artery 24 until the ball 34 is adjacent the source 26 of bleeding.

It is to be understood that the wire 32 is the part of the side branch coagulator 10 which is electrically connected to the source 16 of electrical current. Accordingly, the source 16 can be activated to generate an electrical current pulse. This pulse is conducted through the wire 32 as described above to heat the ball 34, which in turn causes a thrombus to form in the side branch 28 to coagulate tissue and blood near the source 26 of bleeding and thereby stop blood flow from the source 26 of bleeding into the artery 24 of the patient 14.

The details of the structure of the present invention can be seen in reference to Figures 3 through 7. In Figure 3, the wire 32 is shown to include an inner conductor 36 and an insulating layer 38. As shown in Figure 3, the wire 32 can be substantially linear in configuration throughout its length. The diameter of the inner conductor 36 is sufficiently small to permit advancing the wire 32 into narrow body vessels, and sufficiently large to impart enough rigidity to the wire 32 to permit the wire 32 to be advanced through a body vessel by pushing the wire 32. The skilled artisan will accordingly appreciate that the wire 32, given its relatively narrow diameter, can be efficaciously advanced into narrow body vessels, e.g., the side branch 26 shown in Figure 2. On the other hand, the wire 32 retains sufficient axial strength (i.e., rigidity) to permit the wire 32 to be pushed through the guide catheter 30.

The inner conductor 36 is preferably a rigid, electrically conductive wire. Further, the insulating layer 38 is made of any suitable material which insulates the inner conductor 36 from the surrounding environment. In accordance with the present invention, the material used for the insulating layer 38 is a biocompatible material. In one preferred embodiment the insulating layer 38 is made of a polymeric coating, and is deposited on the inner conductor 36 by means well-known in the art.

Figure 3 also shows that a suitable heat generator, such as the ball 34, is attached to the distal end of the wire 32. The ball 34 can be formed integrally with the inner conductor 36 or can be welded or brazed to the inner conductor 36. As contemplated by the present invention, the ball 34 is made of a suitable biologically inert, biocompatible, electrically resistive material, such as stainless steel, that will become energized when electrical current is conducted to it.

The diameter of the ball 34 is sufficiently small to permit positioning the ball 34 in narrow passageways, such as the side branch 26 shown in Figure 2, and to permit relatively rapid heating of the ball 34 when electrical current is conducted to it. On the other hand, the diameter of the ball 34 is sufficiently large to permit the ball 34 to efficiently and rapidly coagulate tissue and blood that surrounds sources of bleeding.

Also, it is to be understood that while the heat generator shown in the Figures has a spherical configuration, other configurations can be used, albeit with different heating and coagulating characteristics. For example, a heat generator (not shown) that has an ovoid shape can be used in place of the ball 34.

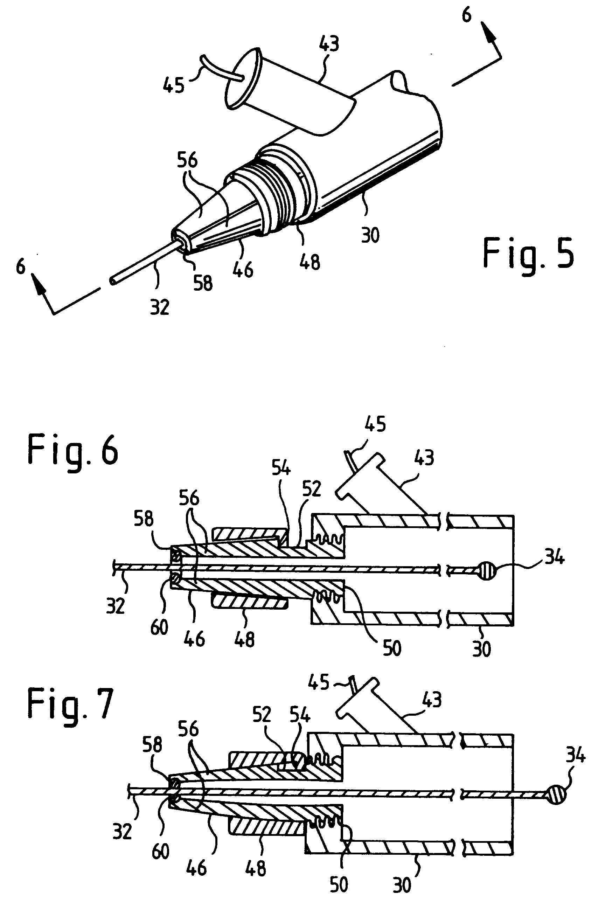

Figure 4 shows that in an alternative embodiment, a resilient member, such as a wire 40, can be provided in place of the wire 32. As shown in Figure 4, the wire 40 is in all essential respects identical to the wire 32, except that the wire 40 has a distal segment 42 that is bent in an arcuate configuration. Stated differently, the distal segment 42 of the wire 40 is materially biased, i.e., is normally curved, into the arcuate shape shown in Figure 4. Additionally, a ball 44 is attached to the distal segment 42 of the wire 40. The skilled artisan will appreciate that by bending or otherwise forming the distal segment 42 in an arcuate shape, the ball 44 of the wire 40 can be positioned adjacent hard-to-reach sources of bleeding simply by rotating the wire 40.

Now referring to Figure 5, the guide catheter 30 is shown in operable engagement the wire 32. It is to be appreciated that the catheter 30 houses the distal segment of the wire 32, including the ball 34, when it is not desired to coagulate blood flow from a source of bleeding. Thus, the catheter 30 shields the patient 14 from the ball 34 to prevent inadvertently applying heat to the patient 14. On the other hand, when it is desirable to coagulate tissue and blood that is near a source of bleeding within a side branch of the patient 14, the wire 32 can be slid within the catheter 30, as more fully disclosed below, to expose the ball 34 to the source 26 of bleeding.

As shown in Figure 5, the guide catheter 30 includes at least one hollow, elongated, relatively thin tube which can be manually advanced through the artery 24 by pushing the proximal segment of the catheter 30. Preferably, the guide catheter 30 has an outer diameter of about one and one-half millimeters to about three millimeters (1.5 - 3.0 mm), to permit inserting the guide catheter into relatively narrow body vessels or cavities. Also, the guide catheter 30 is made of a strong, biocompatible material, e.g., titanium, plastic, or stainless steel.

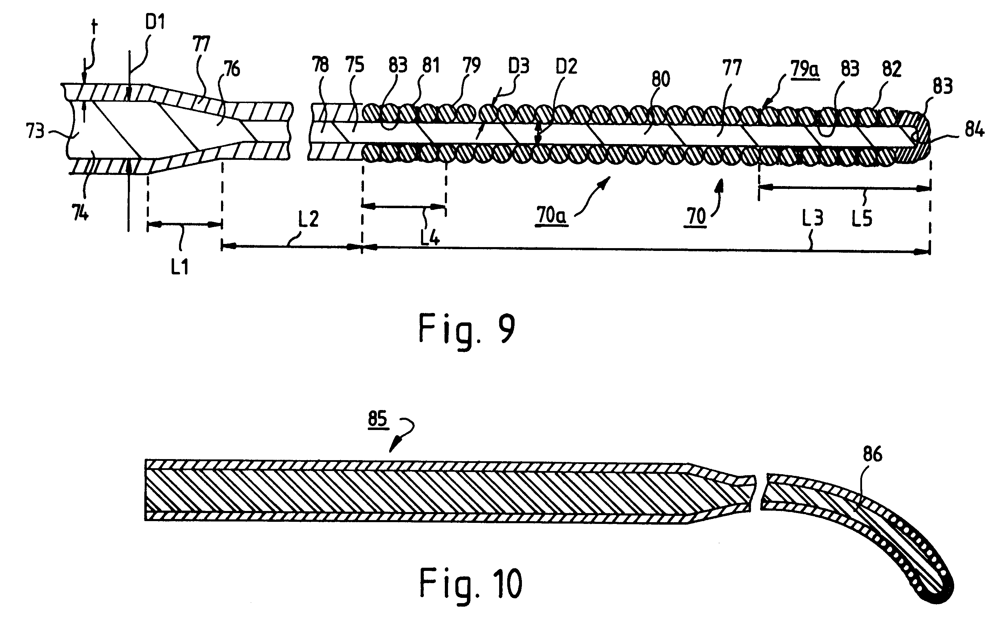

In one presently preferred embodiment, the guide catheter 30 can be one of the catheters that is disclosed in U.S. Patent No. 5,026,383, assigned to the same assignee of the present invention and incorporated herein by reference. Specifically, the cutting catheter of the device disclosed in U.S. Patent No. 5,026,383 can be used as the guide catheter of the present invention. The working length of the catheter disclosed in the above-referenced U.S. Patent is about one hundred fifty centimeters (150 cm). As also disclosed in the above-referenced U.S. Patent, a connector 43 can be connected to the catheter 30 to permit a means by which a fiber optic cable 45 can be positioned within the guide catheter 30. The cable 45 permits the surgeon 12 to view the operating site, to facilitate coagulating blood from the source 26 of bleeding. Alternatively, the guide catheter 30 can be the valvulotome shown in Figure 8 and described more fully below.

Still referring to Figure 5, a means for selectively holding the wire 32 stationary with respect to the guide catheter 30 is shown. In the presently preferred embodiment shown in Figure 5, this means includes a collet 46 and a chuck 48. As shown, both the collet 46 and chuck 48 are coaxial with the guide catheter 30 and surround the guide catheter 30.

The collet 46 is attached to the guide catheter 30 by any suitable means. For example, the collet 46 may be bonded or bolted to the catheter 30. Preferably, however, the distal end 50 of the collet 46 shown in Figure 6 is formed as a threaded luer connection that can engage a complementary luer connection on the guide catheter 30.

Figures 5 and 6 show that the chuck 48 surrounds the collet 46. In accordance with the present invention, the chuck 48 can slide axially over the collet 46. Thus, the chuck 48 is in slidable engagement with the collet 46. To limit the range of axial movement of the chuck 48 relative to the collet 46, i.e., to prevent the chuck 48 from inadvertently sliding off the collet 46, the collet 46 is formed with a groove 52, and a protrusion 54 is formed on the chuck 48 to slidably engage the groove 52. The protrusion 54 can abut the distal wall of the groove 52 to limit distal motion of the chuck 48 relative to the collet 46. Also, the protrusion 54 can abut the proximal wall of the groove 52 to limit proximal motion of the chuck 48 relative to the collet 46.

Figures 5 and 6 further show that the collet 46 includes a plurality of leaves 56. Each of the leaves 56 is materially biased outwardly from the longitudinal axis of the collet 46. A resilient o-ring 58 circumscribes the interior of the collet 46 near the proximal end 60 of the collet 46. Stated differently, the o-ring 58 is in contact with the inner surface of each of the leaves 56. This o-ring 58 is made of a polymeric or other suitable biocompatible, resilient material.

The operation of the collet 46 and chuck 48 can be seen and appreciated in the cross-reference to Figures 6 and 7. Figure 6 shows that the chuck 48 can be moved in a proximal direction on the collet 46 into a "slide" position, wherein the chuck 48 releases the leaves 56 of the collet 46. Thus, when the chuck 48 is in the slide position shown in Figure 6, the collet 46 is not compressed by the chuck 48. Consequently, when the chuck 48 is in the slide position, a clearance exists between the o-ring 58 and the wire 32, so that the wire 32 can be slid freely in the longitudinal direction, i.e., distally or proximally within the catheter 30.

When it is desired to prevent relative motion between a wire 32 and catheter 30, the chuck 48 is moved distally relative to the collet 46 into a "hold" position, shown in Figure 7. In this position, the chuck 48 compresses the leaves 56 of the collet 46 to cause the o-ring 58 to urge against the wire 32 and hold the wire 32 stationary with respect to the catheter 30.

Accordingly, with the wire 32 completely housed within the guide catheter 30 and the chuck 48 in the hold position, the surgeon 12 can advance the guide catheter 30 through the patient 14 until the distal end of the guide catheter 30 is near a source of bleeding. Then, the surgeon 12 can move the chuck 48 to the "slide" position shown in Figure 6 and manually push the rigid wire 32 distally until the ball 34 is no longer housed within the catheter 30. The surgeon 12 manipulates the wire 32 as appropriate to position the ball 34 close to the source 26 of bleeding.

When the ball 34 has been exposed to the source 26 of bleeding, the surgeon 12 moves the chuck 34 to the hold position, shown in Figure 7. The surgeon 12 next depresses the foot pedal 20 (Figure 1) to cause a pulse of electricity to be conducted through the wire 32 and heat the ball 34. The heat from the ball 34 is radiated or conducted into the side branch that contains the source 26 of bleeding, thereby causing blood from the source 26 and tissue that is near the source 26 to coagulate. This coagulation stops the flow of blood from the source 26 of bleeding into the artery 24, without substantially affecting the patency of the artery 24.

After blood flow the source 26 of bleeding to the operating site has been stopped, the surgeon moves the chuck 48 to the position shown in Figure 6 and retracts the wire 30 with ball 34 into the catheter 30. This houses the ball 34 within the catheter 30 to prevent exposing the ball 34 to tissue in the patient 14. The chuck 48 is then moved to the hold position, to hold the distal segment of wire 32 within the catheter 30. The process is repeated as necessary to coagulate other side branches (not shown) or to cauterize or coagulate additional sources of bleeding (not shown).

It is to be appreciated that the wire 40 shown in Figure 4 can be used in the present invention in place of the wire 32. In such an embodiment, when the wire 40 is fully retracted into the catheter 30, the catheter 30 urges against the distal segment 42 of the wire 40. Consequently, the distal segment 42 of the wire 40 is straightened sufficiently to permit the wire 40 to be completely retracted into the catheter 30 by the cooperation of structure between the catheter 30 and wire 40. Thereby, the ball 44 is completely housed within the catheter 30. In this sense, the wire 40 is resilient, i.e., the distal segment 42 is urged out of its at rest configuration when the wire 40 is retracted into the catheter 30. It is to be understood, however, that the wire 40 retains sufficient axial strength to permit pushing the wire 40 distally in the catheter 30.

Accordingly, the wire 40 can be slid distally relative to the catheter 30 to expose the ball 44 to a source of bleeding. When the distal segment 42 clears the catheter 30, the distal segment 42 resiliently resumes its arcuate configuration shown in Figure 4. Stated differently, when the distal segment 42 is free of the catheter 30, the distal segment 42 springs into its normally curved configuration. The wire 40 or, when the chuck 48 is in the hold position, the catheter 30, can be rotated as appropriate to position the ball 44 adjacent hard-to-reach sources of bleeding.

Now referring to Figure 8, an alternate embodiment of the present invention, referred to herein as an electro-ligator, is shown, generally designated 70. As can be appreciated in reference to Figure 8, the electro-ligator 70 can be slidably engaged with the lumen of a catheter-like device. As contemplated by the present invention, the catheter-like device can be, e.g., the Apparatus for In-Situ Cutting of Valves Within Veins disclosed in U.S. Patent No. 5,026,383, assigned to assignee of the present invention and incorporated herein by reference, or the Detachable Tip Optical Valvulotome disclosed in co-pending U.S. patent application serial no. 07/895,090, filed June 8, 1992, assigned to assignee of the present invention and incorporated herein by reference, or, as shown in Figure 8 and disclosed in detail below, the Optical Valvulotome with Pushing Catheter, generally designated 100, which is disclosed in co-pending U.S. patent application serial no. , filed February 19, 1993, assigned to assignee of the present invention and incorporated herein by reference.

Figure 8 shows that the electro-ligator 70 is electrically connected to a cautery connector 71, and the cautery connector 71 is in turn electrically connected to a source 72 of electricity. In the presently preferred embodiment, the source 72 is a Bovie model surgical electrical pulse generator.

Now referring to Figure 9, the details of the electro-ligator 70 can be seen. As shown in Figure 9, the electro-ligator 70 has an elongated, flexible, electrically conductive core 73, and the core 73 includes a cylindrical proximal segment 74 and a cylindrical distal segment 75. It is to be understood that the proximal segment 74 of the core 73 is electrically connected to the source 72 of electricity (Figure 8).

As shown, the proximal segment 74 of the core 73 includes a frusto-conical shaped tapered portion 76 which is faired into the distal segment 75. Preferably, the core 73 is made of 304 stainless steel, and at least part of the distal segment 75 is annealed to one-half normal hardness.

Figure 9 shows that in one presently preferred embodiment, the proximal segment 74 of the core 73 has a diameter D1 of about sixteen-thousandths of an inch (0.016"), while the distal segment 75 of the core 73 has a diameter D2 of about five-thousandths of an inch (0.005"). Preferably, the length L1 of the tapered portion 76 is about three-tenths of an inch (0.3").

As intended by the present invention, an electrically-resistive Teflon® sheath 77 is shrink-wrapped onto the outer surface of the proximal segment 74 of the core 73 and on a portion 78 of the distal segment 75 of the core 73. In the exemplary embodiment shown in Figure 9, the length L2 of the portion 78 of the distal segment 75 which is covered by the sheath 77 is equal to about two and thirty four-hundredths of an inch (2.34").

To ensure that the sheath 77 establishes an adequate electrical insulation barrier when the electro-ligator 70 is to be used in conjunction with a Bovie-model source 72 of electricity to stop blood flow from the side branch of a human saphenous vein, the sheath 77 preferably has a relatively high breakdown potential. More particularly, the breakdown potential of the sheath 77 is such that less than three-tenths of a milliamp (<0.3ma) will flow through the sheath 77 when a voltage potential of twelve hundred volts (1200V) is applied across the sheath 77 in the radial dimension.

To establish a relatively high breakdown potential for the sheath 77, in one preferred embodiment the sheath 77 has a thickness "t" of about three-thousandths of an inch (0.003"), and is made of relatively high-purity Teflon®.

Figure 9 shows that a unifylar close wound coil 79 is disposed around the distal-most portion 80 of the distal segment 75 of the coil 73, and the coil 79 accordingly is in electrical contact with the distal segment 75. Preferably, the coil 79 is made of a suitable biocompatible electro-cautery material, such as 304 stainless steel. In the embodiment shown in Figure 9, the distal-most portion 80 of the distal segment 75 (i.e., the portion of the distal segment 75 around which the coil 79 is disposed) has a length L3 of between about twelve-hundredths of an inch and thirty-hundredths of an inch (0.12"-0.30"), and the length L3 is most preferably about sixteen-hundredths of an inch (0.16"). Further, the coil 79 has a diameter D3 of about four-thousandths of an inch (0.004").

Still referring to Figure 9, the coil 79 has a proximal coil section 81 and a distal coil section 82, and the proximal coil section 81 and distal coil section 82 are welded or brazed to the core 73. Consequently, an electrically heatable brazing or welding bonding material 83 is deposited between adjacent loops of the coil 79 in the proximal and distal coil sections 81, 82, and the bonding material 83 is in thus in electrical contact with the core 73. Preferably, the material 83 has a melting point in excess of one thousand degrees Fahrenheit (1000°F). Together, the coil 79 and material 83 establish an electro-cautery element, generally designated 79a.

Additionally, as shown in Figure 9, the material 83 is deposited on the distal tip 84 of the core 73. In the embodiment shown in Figure 9, the proximal coil section 81 covers a length L4 of the core 73 of about twenty-thousandths of an inch (0.020"), whereas the distal coil section 82 covers a length L5 of the core 73 of about thirty-thousandths of an inch (0.030").

In the operation of the electro-ligator 70, the electro-ligator 70 is advanced through the working channel of the valvulotome 100 (Figure 8). Then, the valvulotome 100 is advanced into the saphenous vein of a patient to cut away valves within the vein while the surgeon views the operating site by means of an optical display system which is operably coupled to the valvulotome 100, as more fully disclosed below. During the valvulotomy procedure, the distal segment 75 of the core 73 is advanced out of the working channel of the valvulotome 100 and into a side branch of the saphenous vein through which fluid to or from the saphenous vein can flow. Fluid flow through the side branches must be stopped, so that once the saphenous vein is arterialized, arterial blood from the saphenous vein cannot invade the venous system through the side branches.

Once the distal segment 75 has been positioned as appropriate within the side branch, the source 72 of electricity is activated to generate a pulse of electricity. The electrical pulse is conducted through the core 73 to the coil 79 and material 83, which has been positioned near or against tissue, to dessicate the tissue, to create acute trauma to the intimal lining of the side branch and thereby induce chronic thrombosis, and to coagulate blood and thereby induce acute thrombosis. As a consequence, fluid flow through the side branch is stopped, i.e., the side branch is occluded by cauterizing the side branch.

With the structure disclosed above, the distal segment, generally designated 70a, of the electro-ligator 70 retains flexibility sufficient to permit the distal segment 70a to be advanced into a relatively small-diameter fragile side branch of a human saphenous vein. Specifically, the relatively small-diameter distal segment 75 of the core 73, with coil 79, is relatively flexible, so that the distal segment 70a of the electro-ligator 70 is consequently flexible.

On the other hand, the distal segment 70a of the electro-ligator 70 has a sufficiently large surface area to limit the density of the current flowing from the distal segment 70a into adjacent tissue, and to consequently permit controllable stoppage of blood flow from the side branch while minimizing unwanted tissue damage. More specifically, by disposing the coil 79 around the distal segment 75 of the core 73, the outer surface area of the distal segment 70a of the electro-ligator 70 is thereby increased, relative to the diameter D2 of the distal segment 75 of the core 73, and the density of current flow from the distal segment 70a into nearby tissue is accordingly less than it would otherwise be without the coil 79.

Now referring to Figures 10-12, alternate embodiments of the electro-ligator of the present invention are shown which have distal segments that are materially biased during the annealing process into arcuate shapes, to facilitate advancing the various distal segments into saphenous vein side branches.

More specifically, Figure 10 shows an electro-ligator 85 which has a distal segment 86 that is materially biased into a gently curved configuration. The distal segment 86 has sufficient flexibility to allow it to straighten and thus slide within a relatively small-diameter lumen. When the distal segment 86 is advanced out of the lumen and is accordingly no longer constrained by the wall of the lumen, the distal segment 86 assumes its arcuate configuration, to permit it to be more easily advanced into a side branch.

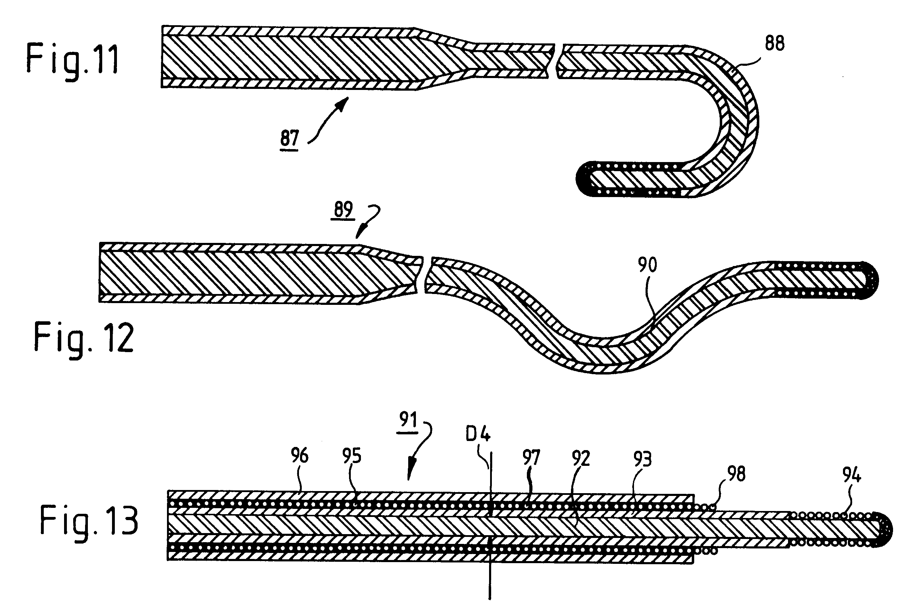

Likewise, Figure 11 shows an electro-ligator 87 having a flexible J-shaped distal segment 88, while Figure 12 shows an electro-ligator 89 having a generally S-shaped distal segment 90. It is to be understood that the electro-ligators 85, 87, 89 are in all other essential respects identical to the electro-ligator 70.

Figure 13 shows a bi-polar electro-ligator, generally designated 91. As shown in Figure 13, the electro-ligator 91 has an elongated cylindrical stainless steel core 92 surrounded by a first Teflon® sheath 93 which is shrink-wrapped thereto. The steel core 92 has a first electro-cautery element 94, which is in all essential respects identical to the electro-cautery element 79a shown in Figure 9, and the first electro-cautery element 94 is brazed or welded to the core 92.

A coil 95 is disposed closely around the first Teflon® sheath 93 to establish a second electro-cautery element, and a second Teflon® sheath 96 is shrink-wrapped around a proximal segment 97 of the coil 95 to insulate the proximal segment 97 from tissue. A distal segment 98 of the coil 95, however, is not covered by the second sheath 96, and a second electro-cautery element is consequently established by the distal segment 98 of the coil 95. Accordingly, the core 92 and the coil 95 can be electrically connected to a bi-polar Bovie current source (not shown) by means well-known in the art to establish a bi-polar electro-ligator 91. The operation of the bi-polar electro-ligator 91 is in all essential respects identical to the operation of the electro-ligator 70 shown in Figure 9.

In the embodiment shown in Figure 13, the core 92 has a diameter D4 of about five-thousandths of an inch (0.005"). Also, each coil is made of four-thousandth of an inch (0.004") diameter steel wire.

Now referring to Figures 8 and 14-18, the details are shown of the valvulotome of the present invention, generally designated 100. As shown, the valvulotome 100 includes a hollow cutting head 102 which is fixedly attached to a hollow catheter 104, as more fully disclosed below. A polyvinylchloride (PVC) "Y" connector 106 is connected to the catheter 104, and an optics tube 108 is connected to the Y connector 106. Also, an extension 110 of the catheter 104 extends through the Y connector 106. It is to be understood that the Y connector 106 is bonded with epoxy to the catheter 104 and optics tube 108.

Figure 8 shows that a hub coupler 112 is connected to the optics tube 108. Preferably, the hub coupler 112 is a Baxter Healthcare Corp, Less Invasive Surgery Division hub coupler. The hub coupler 112 includes a light cable connector 114, and the light cable connector 114 can be engaged with a suitable light source with cable (not shown), such as an American Cystoscope Manufacturers Inc. light source with cable. Further, a video camera system (not shown) can be engaged with a hub 116, for displaying an image of the interior of a blood vessel, as more fully discussed below.

Still referring to Figure 8, a segment 118 of PVC strain relief tubing surrounds the extension 110 of the catheter 104 near the Y connector 106, to prevent kinking of the extension 110. Also, a fitting 120 is connected to the extension 110, and a segment 122 of strain relief tubing surrounds the extension 110 near the fitting 120. Preferably, the fitting 120 is a stepless tapered luer fitting, made, for example, by Medical Disposables, Inc.

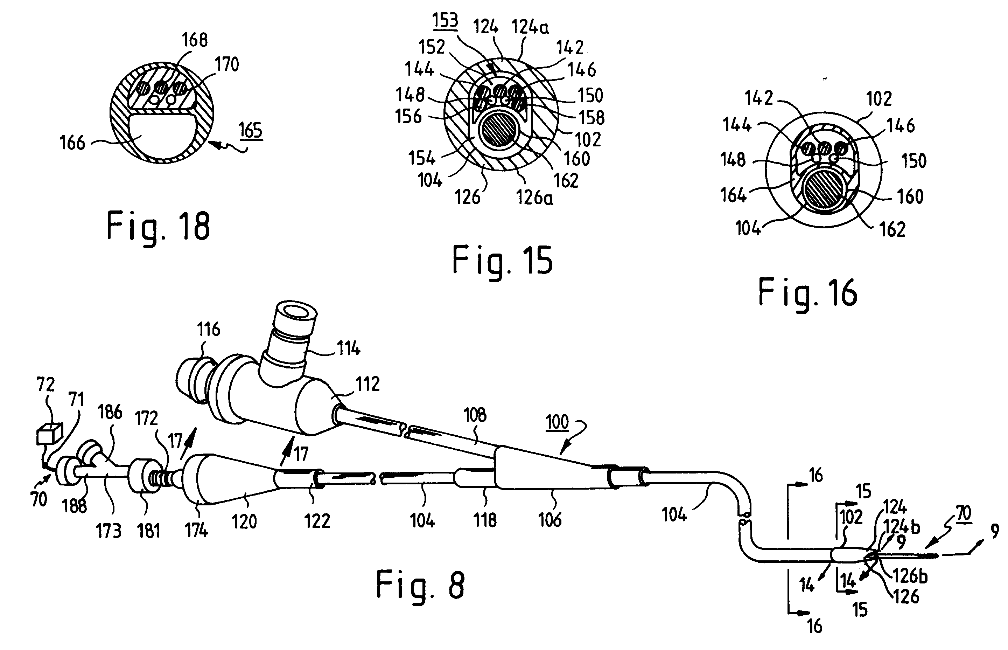

Now referring to Figures 14 and 15, the details of the catheter 104 with cutting head 102 can be seen. As shown best in Figure 14, the cutting head 104 is a hollow unitary structure, and is preferably made of stainless steel, hard plastic, or other suitable biocompatible material.

Figure 14 shows that the cutting head 102 has a pair of opposed blades 124, 126, each of which has a respective rounded outer surface 124a, 126a to generally conform to the contour of a blood vessel (not shown) on which valvulotomy is to be performed. Each rounded outer surface 124a, 126a has a respective parabola-shaped sharpened cutting edge 124b, 126b, and the edges 124b, 126b are contiguous so that together they define a U-shaped opening 128 having a radius of curvature R1 of about fourteen-thousandths of an inch (.014") at its closed end. Stated differently, each cutting edge 124a, 126a has a respective distal tip 130, 132, each of which is positioned at the apex of a parabola-shaped cutting edge 124b, 126b.

The tips 130, 132 of the blades 124, 126 are spaced from each other, to establish an open distal end of the cutting head 102. In other words, a light passageway is established from the open distal end of the cutting head 102 to the interior sides of the blades 124, 126 and adjacent regions within the blood vessel through which the cutting head 102 is advanced.

Figure 14 further shows that in longitudinal cross-section, the outer surfaces 124a, 126a of the blades 124, 126 taper slightly inwardly toward the distal tips 130, 132 of the blades 124, 126. Consequently, an angle α of about six and three-tenths degrees (6.3 degrees) is established between the outer surfaces 124a, 126a of the blades 124, 126 and the longitudinal axis 134 of the cutting head 102. On the other hand, as shown in Figure 14, the inner surfaces 124c, 126c of the blades 124, 126 are essentially cylindrical.

Still referring to Figure 14, each of the blades 124, 126 has a length L1 of about one hundred fifty-thousandths of an inch (.150"), and the blades 124, 126 are integral with a hollow cylindrical sleeve 136 of the cutting head 102. The sleeve 136 has an outer surface 136a, and the outer surfaces 124a, 126a of the blades 124, 126 are flush with the outer surface 136a of the sleeve 136 at the juncture 138 that is established between the blades 124, 126 and sleeve 136.

Figure 14 shows that the sleeve 136 has an outer diameter D3 of about two and eight-tenths millimeters (2.8mm) and a length L2 of about two-tenths of an inch (0.2"). An annular seating surface 140 is established between the blades 124, 126 and the sleeve 136.

Continuing with the description of Figure 14, the catheter 104 is shown coaxially engaged with the cutting head 102, with the distal end of the catheter 104 abutting the seating surface 140 of the cutting head 102. As shown, the catheter 102 fits snugly within the sleeve 136 of the cutting head 102. Preferably, the catheter 104 is bonded with epoxy to the cutting head 102.

As shown in cross-reference to Figures 14 and 15, the catheter 104 includes a plurality of optical illumination fibers and an optical imaging fiber. Preferably, the catheter 104 includes an image fiber 142 having a diameter of twenty five-thousandths of an inch (.025") and two illumination fibers 144, 146, each having a diameter of about twenty-thousandths of an inch (.020"). Also, the catheter 104 includes two illumination fibers 148, 150, each having a diameter of about ten-thousandths of an inch (.010").

An epoxy material 152 is deposited between the fibers 142, 144, 146, 148, 150 in a distal segment 154 of the catheter 104, i.e., the epoxy is deposited in the segment of the catheter 104 that is held within the sleeve 136 of the cutting head 102. Together, the fibers 142, 144, 146, 148, 150 and epoxy material 152 establish an endoscope, generally designated 153, and a 2.3mm endoscope such the type made by Neuro Navigational Corp. of Costa Mesa, California can be used as the endoscope 153 of the present invention.

As can be appreciated in cross-reference to Figures 14 and 15, the distal end of the endoscope 153 is substantially co-planar with the open distal end of the catheter 104. Thus, the endoscope 153 is mounted within the catheter 104 in light communication with the light passageway established by the cutting head 102 for generating an image of the interior sides of the blades 124, 126 and the adjacent regions within the blood vessel undergoing valvulotomy.

As shown in Figure 15, to urge the distal segments of the illumination fibers 144, 146, 148, 150 near and preferably against the image fiber 142, two rigid spacers 156, 158 are positioned in the catheter 104. Consequently, the imaging capability of the endoscope of the present invention is enhanced, as compared to an endoscope wherein the distal segments of the illumination fibers are not urged against the distal segment of the image fiber. In one presently preferred embodiment, the spacers 156, 158 are cylindrical segments of optical fibers having respective diameters of twenty-thousandths of an inch (.020").

The catheter 104 is preferably formed by extrusion processes well-known in the art, and a cylindrical working channel 160, shown in Figures 14 and 15, is established during the extrusion process. A guide wire 162 can be slidably disposed in the working channel 160, so that the catheter 104 can be pushed into a blood vessel over the wire 162. It will be appreciated by the skilled artisan that the working channel 160 can also be used for irrigation or aspiration of the surgical site. Also, the electro-ligator 70 (Figure 8) can be slidably engaged with the working channel 160, as discussed above.

Referring now to Figure 16, the proximal segment 164 of the catheter 104 does not include epoxy or spacers to urge the optical fibers 142, 144, 146, 148, 150 toward each other. Instead, the fibers 142, 144, 146, 148, 150 are radially constrained only by the wall of the catheter 104, and are not bound together. Consequently, the fibers 142, 144, 146, 148, 150 are permitted to bend relatively easily when the catheter 104 is bent during valvulotomy.

Recall that in the embodiment disclosed above, the working channel 160 is cylindrical, i.e., the working channel 160 is round in cross-section. Briefly referring to Figure 18, however, an alternate embodiment of the valvulotome of the present invention, generally designated 165, is shown which has an enlarged (compared to the working channel 160) working channel 166 that has a semi-circular cross-section. As shown, the valvulotome 165 has an image fiber 168 and a plurality of illumination fibers 170, but owing to the size and shape of the working channel 166, no spacers are required in the valvulotome 165. Instead, the distal segments of the illumination fibers 170 are urged against the distal segment of the image fiber 168 by the wall of the working channel 166.

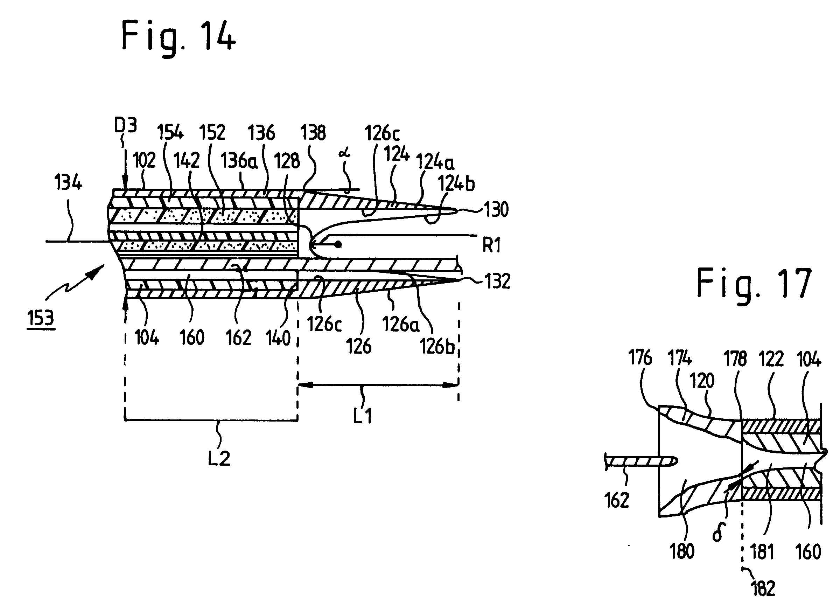

Now referring to Figure 17, the details of the stepless tapered luer fitting 120 can be seen. As shown in Figure 17, the fitting 120 includes a hollow threaded luer element 172 configured for engaging a complementary luer fitting of a surgical device (not shown). Also, the luer fitting 120 includes a hollow body member 174 that has a proximal end 176 and a distal end 178, and the body member 174 defines a passageway 180. Figure 17 shows that the passageway 180 is generally tapered inwardly from the proximal end 176 to the distal end 178 of the body member 174.

Figure 17 further shows that the working channel 160 of the extension 110 of the catheter 104 is contiguous to the passageway 180. As shown, a proximal portion 181 of the working channel 160 is tapered outwardly, distally to proximally, such that the diameter of the working channel 160 at the plane of interface with the passageway 180, indicated by a line 182, is greater than the diameter of the passageway 180 at the plane of interface.

Accordingly, the skilled artisan will appreciate that the guide wire 162 or electro-ligator 70 can be advanced into the passageway 180 of the stepless luer fitting 120 and into the working channel 160 of the catheter 104 without encountering any structure against which the guide wire 162 can undesirably abut. In other words, the guide wire 162 can easily be engaged with the working channel 160 of the catheter 104, without "hanging up" the guide wire 162 on structure which is perpendicular to the direction of advancement of the guide wire 162.

In the operation of the valvulotome 100, reference is made to Figures 8 and 14-18. The guide wire 162 is initially slidably engaged with the working channel 160 of the catheter 104 by advancing the guide wire 162 into and through the passageway 180 of the stepless luer fitting 120. Preferably, the guide wire 162 is advanced through the working channel 160 until a portion of the guide wire 162 projects beyond the cutting head 102.

Then, an incision is made into the blood vessel on which valvulotomy is to be performed, and the guide wire 162 is advanced as appropriate into the blood vessel. Next, the catheter 104 with cutting head 102 is pushed or otherwise advanced into the blood vessel over the guide wire 162, in a direction opposite normal blood flow through the vessel.

As the catheter 104 is advanced into the blood vessel, the cutting head 102 cuts through venous valves within the vessel. The operator of the valvulotome 100 can view the interior of the blood vessel by viewing the image of the blood vessel transmitted through the image fiber 142 and displayed on a nearby endoscopic video monitor. Consequently, the operator can rotate the catheter 104 to orient the blades 124, 126 as appropriate to cut through venous valves.

In accordance with the present invention, the configuration of the cutting head 102 described above facilitates valvulotomy within the blood vessel, while minimizing the risk of unintentionally gouging the wall of the blood vessel.

In addition, the cauterizer of a device such as the Side Branch Coagulator described in co-pending U.S. Patent application Serial No. 07/843,634, filed February 28, 1992, or the electro-ligator 70 disclosed above, can be slidably engaged with the working channel 160 of the catheter 104 to cauterize side branches of the blood vessel undergoing valvulotomy.

## Claims

1. An electro-ligating system for blocking fluid flow through a blood vessel by cauterizing the vessel comprising:
a source of electricity capable of generating electrical pulses;
an elongated flexible metal core having a distal segment, a distal end, and a proximal segment, the core being advanceable into the blood vessel, the core being electrically connected to the source of electricity;
an electrically insulative sheath shrink-wrapped around a substantial portion of the proximal segment of the core; and
a metallic coil disposed around a substantial portion of the distal segment of the core in electrical contact therewith, wherein the source of electricity can be selectively activated to energize the coil and thereby block fluid flow through the blood vessel by cauterizing the blood vessel.

2. The electro-ligator of Claim 1, wherein the distal segment of the core is biased into a predetermined arcuate shape.

3. The electro-ligator of Claim 1 or 2, further comprising an outer coil disposed around the sheath and connected to the source of electricity to establish a bipolar electro-cautery element.

4. The electro-ligator of any of Claim 1 to 3, further comprising a catheter-like apparatus defining a lumen, wherein the core is slidably disposed within the lumen.

5. The electro-ligator of any of Claim 1 to 4, wherein less than about three-tenths of a milliampere (0.3ma) flows through the sheath when at least twelve hundred volts (1200V) is applied across the sheath.

6. A method for cauterizing the side branch of a human saphenous vein to substantially stop fluid flow through the side branch, comprising the steps of:
(a) providing a device comprising:
an electrical conductor having a distal segment biased into a predetermined configuration and a proximal segment, the distal segment being configured to facilitate advancing the distal segment into the side branch;
an electrically insulative sheath surrounding the proximal segment of the electrical conductor; and
an electro-cautery element attached to the distal segment of the electrical conductor for becoming energized when electricity is applied to it;
(b) electrically connecting the device to a source of electricity;
(c) optionally advancing at least the electro-cautery element of the device into the side branch of the saphenous vein; and
(d) activating the source of electricity to energize the electro-cautery element to cauterize the side branch of the saphenous vein, such that blood flow from the side branch into the saphenous vein is substantially stopped.

7. The method of Claim 6, further comprising the step of slidably engaging the device with a valvulotomy apparatus.

8. A valvulotome for cutting venous valves in a blood vessel and engageable with a viewing scope for generating an image of the interior sides of a portion of the valvulotome and the adjacent regions within the vessel to facilitate rotation of the valvulotome as appropriate to cut valves within the vessel, comprising:
a catheter sufficiently flexible to follow the contour of the blood vessel when passed through it and sufficiently axially rigid to permit the catheter to be pushed through the vessel, the catheter having a distal segment; and
a cutting head attached to the distal segment of the catheter, the cutting head comprising a pair of cutting blades having sharp cutting edges and being shaped to engage and cut through the venous valves when the cutting blades are aligned therewith, the blades having exterior sides which are contoured to generally follow the curvature of the interior walls of the blood vessel, the blades being disposed relative to the distal segment of the catheter to provide a light passageway from the open distal end to the interior sides of the blades and adjacent regions within the vessel, wherein the viewing scope is mounted within the catheter in light communication with the light passageway.

9. The valvulotome of Claim 8, wherein each said cutting blade has:
an external contour, viewed in a plan direction perpendicular to the major outer surface of the blade, which has a rounded tip extending in a parabola-like shape to blend with a cylindrical outer surface of the cutting head; and
a cutting edge extending from the tip, the cutting edges of the cutting blades together defining a generally U-shaped opening.

10. The valvulotome of Claim 8, further comprising a stepless tapered luer fitting connected to the catheter to facilitate sliding a wire into the catheter.
